# EUROPEAN PATENT APPLICATION

(11) **EP 1 657 549 A1**
(43) Date of publication of application: **17.05.2006**
(21) Application number: 05257008.2
(22) Date of filing: 14.11.2005
(51) Int. Cl.: G01N 33/543, G01N 21/55

(54) **Apparatus and method for facilitating molecular interaction measurements**

(30) Priority: 12.11.2004 US 987991
(71) Applicant: Agilent Technologies, Inc., Palo Alto, CA 94306 (US)
(72) Inventor: Wilson, William H., Newark, DE 19711 (US); Giffin, Kristin M., Bel Air, MD 21014 (US)
(74) Representative: Ellis, Katherine Elizabeth Sleigh

(57) **Abstract**

A method of facilitating biomolecular interaction measurements includes attaching an interaction species fluidic router (300) to a substrate (305). A set of interaction species samples is routed through the interaction species fluidic router (300) to produce a coated sensor surface (306) with multiple reaction zones corresponding to the interaction species samples. The interaction species fluidic router (300) is then removed from the substrate. A multi-sample fluidic router (310) is then connected to the substrate (305). A set of fluidic samples is then directed through the multi-sample fluidic router (310) to the multiple reaction zones (308). Optical signals corresponding to the resultant biomolecular interactions are then collected. The optical signals are subsequently processed to characterize the biomolecular interactions.

## Description

This invention relates generally to molecular interaction measurements and apparatus and method for facilitating molecular interaction measurements. More particularly, this invention relates to a technique for facilitating the application of multiple fluidic samples to multiple interaction species.

Molecular interactions (e.g., such as interactions between biomolecules) can be measured using a variety of systems. For example, two or more species can be brought together in a suitable interaction environment. The interaction of discrete species or lack of interaction may alter some property of the species or the system. This alteration can then be measured to produce a representative interaction signal. Often, optical systems are used to detect the interaction molecular species. Optical systems typically share a number of common components including a light source to generate a light beam, and optics to direct the light beam to a sample analysis site, such as a flow template. The flow template has an interaction species and is configured to route a fluidic sample over the interaction species. When the light beam interacts with the flow template, a deflected light beam is produced. The properties of the deflected light beam may be used to characterize the biomolecular interactions occurring between the fluidic sample and the interaction species. A detector captures the deflected light beam. The detector typically converts the captured optical signal to a corresponding electrical signal, which is processed by a signal processor to identify biomolecular interactions using well-known techniques.

The biomolecular and molecular interactions occurring between the fluidic sample and the interaction species are sometimes characterized through the use of surface plasmon resonance. Surface plasmon resonance arises when incident light on a free electron metal, such as gold, couples to the electrons, and is dissipated. The reflected light shows a minimum at the angle or wavelength where the light couples to the free electron metal. On the opposite side of the free electron metal, changes in the local refractive index alter the angle or wavelength of light that couples to the metal. Consequently, the surface plasmon resonance sensor detects changes in the local refractive index of the metal surface. A channel is formed with the gold treated glass substrate to form a flow template. If an interaction species is immobilized on the surface of the metal, the minimum in reflected light will shift accordingly. The signal can now be re-zeroed for use as a biomolecular or molecular interaction device.

When molecules in the fluidic sample bind to the gold sensor surface, the concentration and therefore the refractive index at the surface changes and a surface plasmon resonance response is detected. Plotting the response against time during the course of an interaction provides a quantitative measure of the progress of the interaction. This plot is sometimes called a sensorgram.

In such systems, the light is not absorbed by the sample. Instead, the light energy is dissipated through surface plasmon resonance in the gold film. Thus, the light used to detect interaction processes never enters the sample.

Surface plasmon resonance has the advantage that the sample under test does not have to be labeled with a detection moiety in order to produce a signal. This "label-free" detection allows the sample to be chemically unmodified and therefore the sample preserves its structural conformation. This also circumvents the problem of label attachment chemistries and uniform labeling artifacts seen in systems that rely on detection labels. Other examples of "label-free" detection include grating-coupled surface plasmon resonance, reflectometric interference spectroscopy, coupled planar waveguide techniques, and a variety of other approaches.

One shortcoming associated with these systems is that they make a limited number of interaction measurements simultaneously. For example, in some commercial systems, the instruments can make 2 to 4 interaction measurements in parallel. Typically, one of these measurements is used for background subtraction, limiting the number of measurements to 1 to 3. The limited number of sensing zones, however, does allow the system to make high-resolution measurements of binding and provide accurate kinetic data.

Another problem with prior art systems is that the fluidic handling systems will only permit a single sample to be injected at a time. This severely limits throughput. With the growing interest in understanding protein interaction, the need for parallel sample processing and multiple interaction measurements is increasing.

There are also limitations associated with the formation of interaction species on a flow template. One approach is to pump the interaction species sample over the detection zones. Linking chemistry is then used to immobilize the molecules. The advantage of this approach is that the detection system can be used to monitor the surface loading of the interaction species in real time. This allows the user to determine how much reagent was present during the interaction experiments. In addition, this approach allows comparison of loading between experiments for better correlation. However, this process requires several steps and consumes a substantial amount of instrument time to prepare the surface for the actual biomolecular interaction measurements.

To address these limitations, a printer can be used to form interaction zones on a substrate. Unfortunately, this approach requires the purchase of an expensive printer and associated costly infrastructure to use, support and maintain the printer. In addition, since the deposition of the interaction species is performed offline, there is no way to accurately measure the amount of interaction species that is loaded onto the surface. For completely relative interaction measurements between the reaction zone spots, the system is able to provide useful data. However, for absolute measurements, such as kinetic data, the system has difficulties in providing accurate information. In some commercial devices, the grating coupled approach and the charge coupled device detector do not have sufficient resolution or sensitivity to measure low molecular weight (e.g., less than 1000 daltons) species. Furthermore, the flow cell design for the system has problems since it will not efficiently sweep the sample across the sensor surface. These devices have an expansion zone from the small fluidic channel to the sensor surface and then a funnel to a fluidic exit. This results in turbulence (e.g., eddies) in the flow template that prevents useful kinetic data collection. Still another significant limitation of prior art systems of this type is that they are designed to analyze a single sample at a time.

In view of the foregoing, it would be highly desirable to provide an improved technique for biomolecular interaction measurements. In particular it would be highly desirable to provide an improved flow template to facilitate biomolecular interaction measurements.

According to a first aspect of the present invention, there is provided a method as specified in claim 1.

The invention includes a method of facilitating biomolecular interaction measurements. In one aspect, an interaction species fluidic router is attached to a substrate. A set of interaction species samples is routed through the interaction species fluidic router to produce a coated sensor surface with multiple reaction zones corresponding to the interaction species samples. The interaction species fluidic router is then removed from the substrate. A multi-sample fluidic router is then connected to the substrate. A set of fluidic samples is then directed through the multi-sample fluidic router to the multiple reaction zones. Optical signals corresponding to the resultant biomolecular interactions are then collected. The optical signals are subsequently processed to characterize the biomolecular interactions.

In one embodiment, the invention also includes a method of facilitating biomolecular interaction measurements by routing a set of interaction species samples across a sensor surface to provide a coated sensor surface with multiple reaction zones corresponding to the interaction species samples. A set of fluidic samples is directed to the multiple reaction zones to generate a set of optical signals. The optical signals are collected at a two dimensional sensor. The optical signals are processed to identify biomolecular interactions.

According to a second aspect of the present invention there is provided a fluidic system as specified in claim 7.

The invention also includes a fluidic system kit. The kit includes an interaction species fluidic router to route a set of interaction species samples across a sensor surface to render a coated sensor surface with multiple reaction zones corresponding to the interaction species samples. A multi-sample fluidic router adapted to route a set of fluidic samples to the multiple reaction zones is included in the kit. A set of instructions to sequentially use the interaction species fluidic router and the multi-sample fluidic router to facilitate the measurement of biomolecular interactions is also enclosed with the kit.

The invention also includes a substrate with multiple reaction zones. A multi-sample fluidic router simultaneously routes a set of fluidic samples to the multiple reaction zones of the substrate.

Unlike prior art devices, the flow template (e.g., multi-sample fluidic router) of the invention supports massively parallel operations, including parallel analyses of multiple fluidic samples with multiple reaction zones. In one aspect, the invention also facilitates the preservation of accurate kinetic measurements by minimizing turbulence (e.g., eddies and other flow distortions). In another aspect, the invention allows the real-time monitoring of the amount of interaction species immobilized on the surface. Advantageously, multiple reaction zones may be formed simultaneously to reduce the time spent preparing the interaction surface. Finally, in a further aspect, the invention also results in cost savings because the technology can be implemented without expensive chip preparation equipment, such as a printer.

The invention is more fully appreciated in connection with the following detailed description of preferred embodiments described by way of example only with reference to the accompanying drawings, in which:
FIGURE 1 illustrates a biomolecular interaction measurement system configured in accordance with an embodiment of the invention.
FIGURE 2 illustrates processing operations associated with an embodiment of the invention.
FIGURE 3A illustrates the use of components of the invention in accordance with the operations of Figure 2.
FIGURE 3B illustrates the use of alternate components of the invention in accordance with the operations of Figure 2.
FIGURE 4 illustrates an embodiment of the invention utilizing horizontal flow input and output, in contrast to the vertical flow input and output of the previous embodiments. This illustrates that vertical flow inputs can be united with horizontal flow connections to increase the number of interactions measured per sample.
FIGURE 5 is a perspective view of a concentric tube configuration utilized in accordance with an embodiment of the invention.
FIGURE 6 is a side view of a concentric tube configuration utilized in accordance with an embodiment of the invention.
FIGURE 7 is a top view of a vertical tube fluid delivery system with a horizontal common drain for the interaction zones.
Like reference numerals refer to corresponding parts throughout the several views of the drawings.

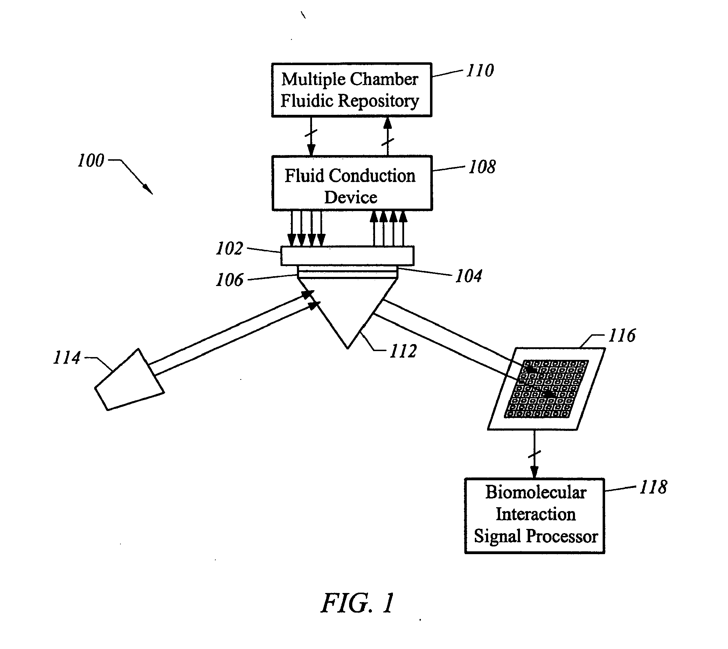

Figure 1 illustrates a biomolecular interaction measurement system 100 configured in accordance with an embodiment of the invention. The system 100 includes at least one fluidic router 102. As will be discussed below, a variety of fluidic routers may be used to implement various operations of the invention. The fluid router 102 interfaces with a film (e.g., a gold film) 104 positioned on a transparent substrate 106 (e.g., glass). In one embodiment of the invention, the fluid router interfaces directly with a prism. In one aspect of the invention, a fluid conduction device 108 delivers fluids to and from the fluidic router 102 using known pumping techniques or other techniques for moving fluid in microfluidic channels (e.g., using electroosmotic or electrokinetic forces). In one embodiment, the fluid conduction device 108 is connected to a multiple chamber fluid repository 110, which stores various fluids utilized in accordance with the invention, as described below. In certain aspects of the invention, the multiple chamber fluid repository may comprise or be interfaced to an industry-standard microtiter plate (e.g., such as a 96-well plate).

Biomolecular interactions produced by the system 100 are processed through the use of a light source 114 that is directed toward one or more optical devices 112. In this embodiment of the invention, a single prism 112 is used as the optical device. Reflected light from the film is collected at a two dimensional sensor 116. The sensor 116 has detection regions corresponding to the locations of biomolecular interaction sites. The collected optical signal is processed by a biomolecular interaction signal processor 118 using standard techniques. By way of example, the biomolecular interaction signal processor 118 may be a general-purpose computer configured with standard software to process the optical signals.

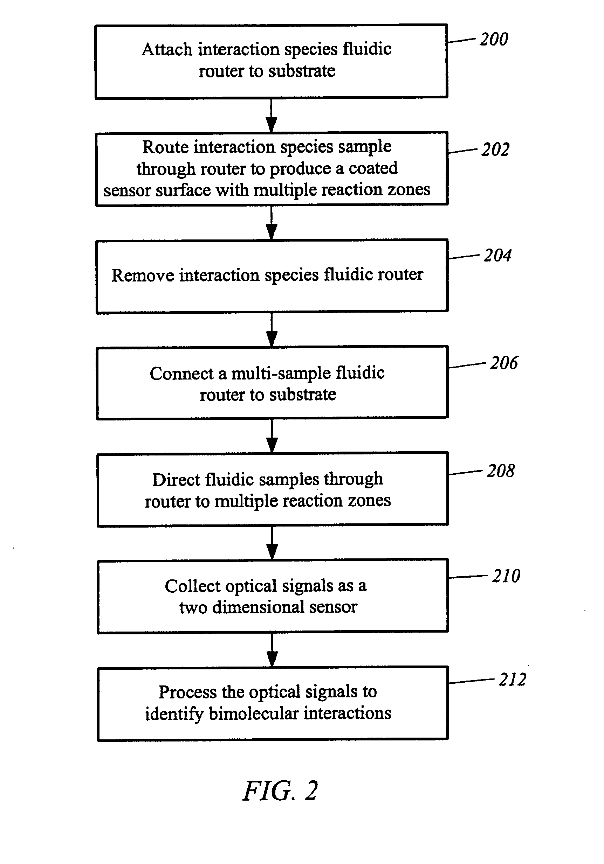

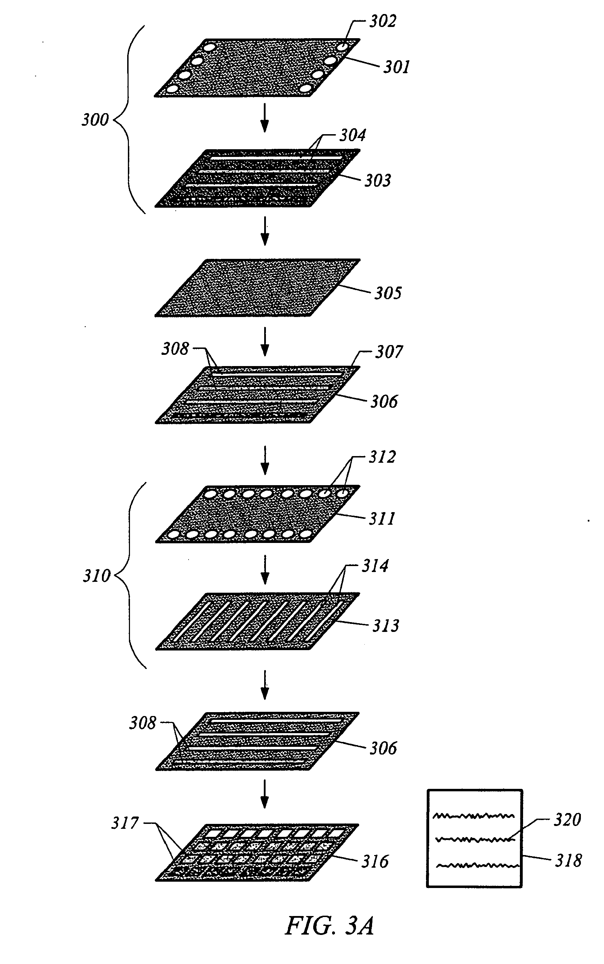

The operation of the system 100 of Figure 1 is more fully appreciated with reference to Figure 2. Figure 2 illustrates processing operations associated with an embodiment of the invention. The first processing operation is to attach an interaction species fluid router to a substrate (200). As implied by its name, the interaction species fluid router is a fluid router adapted to route an interaction species over a substrate (e.g., a transparent substrate with a gold film). Figure 3A illustrates such a substrate 305. The figure also illustrates an interaction species fluid router 300 interfacing with an interaction species port array 301 defining a set of ports 302. The ports 302 are aligned with a set of channels 304 formed in an interaction species channel array 303. The interaction species port array 301 and the interaction species channel array 303 are sandwiched together to form a routing mechanism with four separate channels. The port array 301 and channel array 303 may be formed as a single integral device or as discrete components that are attached to one another.

Interaction species samples are then routed through the router to produce a coated sensor surface with multiple reaction zones (202). For example, four separate interaction species from the multiple chamber fluidic repository 110 may be pumped, using fluid conduction device 108, into the four ports on the left side of the port array 301, traverse through the four channels of the array 303, and exit through the four ports on the right side of the port array 301.

Since the interaction species fluid router 300 is attached to the substrate 305, the pumped interaction species move across the sensor surface in the described orientation. The interaction reagents are immobilized across the sensor surface and the instrument can measure the amount of immobilization across each channel. This provides the user with the immobilization data needed to account for kinetic data. This operation produces a coated sensor surface with multiple reaction zones. The reaction zones can include proteins, nucleic acids, peptides, small molecules, and the like. For example, a first reaction zone may include a first antibody, a second reaction zone may include a second antibody, a third reaction zone may include a mixture of the first antibody, the second antibody, and a third antibody.

Figure 3A illustrates an aspect of the invention, in which the substrate 306 is configured as a coated sensor surface 307 with multiple reaction zones 308. In this embodiment, each reaction zone 308 corresponds to the respective interaction species that it was exposed to. The locations of the reaction zones 308 correspond to the locations of the channels 304 of the fluidic router 300.

Those skilled in the art will appreciate that the invention provides a relatively easy and inexpensive way to form multiple reaction zones on a substrate and does not require an expensive printer to form reaction zones.

Returning to Figure 2, in one embodiment, the interaction species fluidic router is then removed from the substrate (204). A multi-sample fluidic router is then connected to the substrate (206). Figure 3A illustrates a multi-sample fluidic router 310 interfaced with a fluid port array 311, which includes a set of fluid ports 312. In this example, the multi-sample fluidic router 310 also includes a fluidic channel array 313 defining fluid channels 314. The fluid port array 311 and fluidic channel array 313 may be formed as a single integral device or as discrete components that are attached to one another.

The multi-sample fluidic router 310 is attached to the previously prepared substrate 306. Observe that the fluid channels 314 have an orthogonal orientation relative to the multiple reaction zones 308.

The next operation illustrated in Figure 2 is to direct fluidic samples through the fluidic router 310 to the multiple reaction zones (208). This results in a set of reaction sites and does not require the use of a printer. Figure 3A illustrates substrate 316 with multiple reaction sites 317. In one embodiment, each reaction site 317 corresponds to the intersection of a channel 304 from the interaction species channel array 303 and a channel 314 of the fluidic channel array 313. In another embodiment, the invention facilitates the simultaneous application of multiple fluidic samples (e.g., 8 in this example) to multiple reaction sites (e.g., 4 in this example), permitting parallel processing of multiple fluidic samples.

The information (e.g., biomolecular interactions) at the reaction sites may be processed using any number of techniques. For example, the configuration of Figure 1 may be used to collect optical signals at a two-dimensional sensor (operation 210 of Figure 2). The optical signals are then processed to identify biomolecular interactions (operation 212 of Figure 2). Any number of known signal processing techniques may be used.

Thus, Figure 2 illustrates processing operations associated with an embodiment of the invention, while Figure 3A illustrates various devices that may be used to implement these operations. The components of Figure 3A may be combined into a kit for commercial sale. The kit may include an instruction sheet 318 including a set of instructions 320 consistent with those outlined in Figure 2.

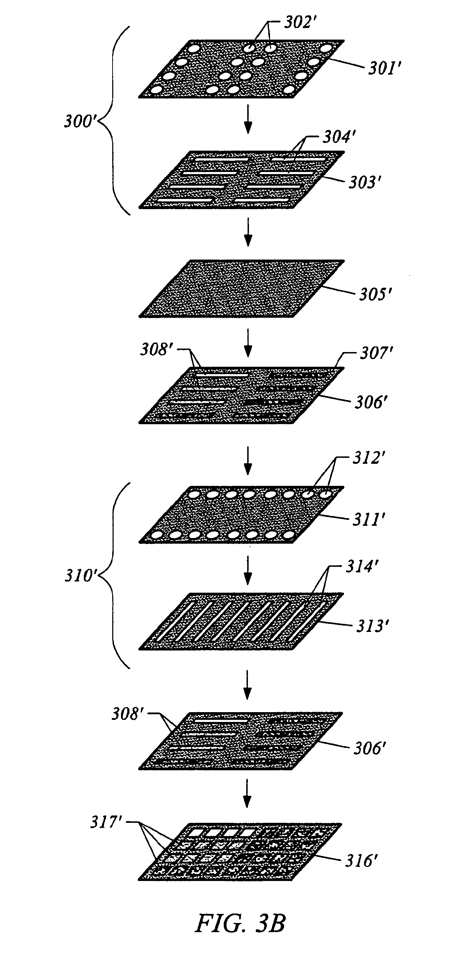

The operations of Figure 2 may be utilized in accordance with any number of interaction species fluidic routers 300 and multi-sample fluidic routers 310. For example, Figure 3B generally corresponds to Figure 3A, however, in Figure 3B the interaction species fluidic router 300 has a compound configuration allowing for eight separate channels 304', which result in eight separate reaction zones 308' formed on substrate 306'. In this example, if a multi-sample fluidic router 310' of the type used in Figure 3A is employed, then multiple reaction sites 317' can be formed. However, in this example, the reaction sites may result from the application of eight separate fluids to the substrate 306'. Thus, eight separate fluids are applied to eight separate reaction zones, demonstrating the multiplexing and scalability advantages associated with the invention.

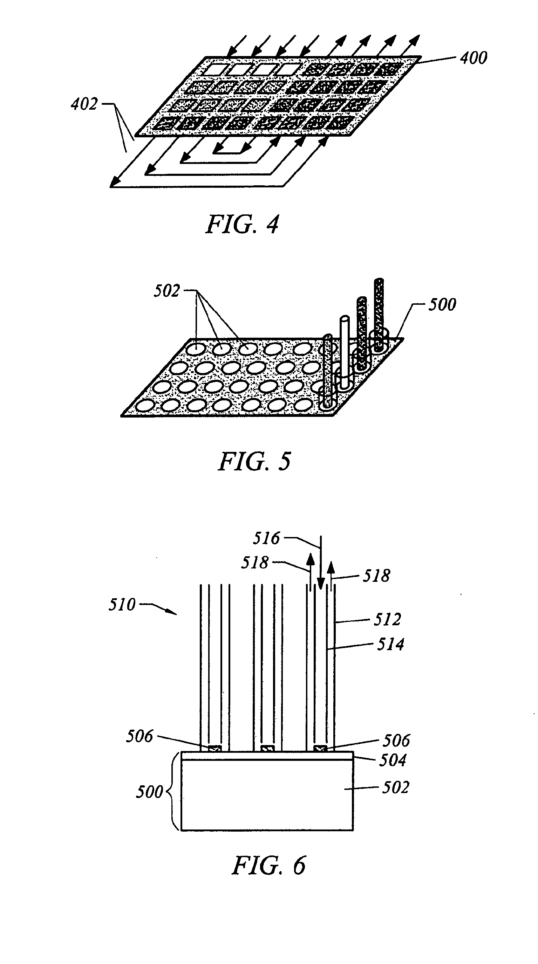

Those skilled in the art will appreciate that the operations of Figure 2 may be used in connection with any number of fluidic router configurations. For example, the embodiments of Figures 3A and 3B relied upon vertical ports for ingress and egress fluidic paths. Horizontal ports may also be used in accordance with embodiments of the invention. Figure 4 illustrates a multi-sample fluidic router 400 with horizontal ports 402. This configuration allows four separate fluidic samples to be applied against eight separate reaction zones (e.g., if the substrate 306'1 of Figure 3B is used).

The techniques of the invention can be used to create reaction zones of arbitrary complexity. Similarly, the techniques of the invention can be used to apply disparate fluidic samples to every reaction zone of a substrate. By way of example, the disparate fluidic samples may have different molecules, different concentrations, different pH levels, and combinations thereof.

Figure 5 illustrates a perspective view of substrate 500 with M interaction zones, where M is the number of reaction zones in the vertical dimension of the substrate. Such a configuration allows up to M different samples to be applied to M different interaction zones simultaneously. In some aspects, fluidic samples comprising differing concentrations of molecules (e.g., biomolecules) may be applied to each reaction zone (for example, to obtain dose-response curves).

Figure 6 is a cross-sectional view of a portion of the system of Figure 5. Figure 6 illustrates the substrate 500 including a film 504 formed on a transparent base 502. Reaction zones 506 are formed on the film 504. Positioned over the substrate 500 is a multi-sample fluidic router. Surrounding each reaction zone 506 is a set of concentric tubes 512 and 514, which form an ingress path 516 and an egress path 518.

In this configuration, flow is directed through the inner concentric tube 514 and flows out and over the surface of the reaction zone 506. The outer concentric tube 512 then conducts the flow away from the surface in an efficient fashion. To assist in the fluid clearance, reduced pressure may be used in the fluid conduction device 108. Alternately, various configurations utilizing gravity may also be used. Observe that this embodiment provides a high degree of throughput as completely different reaction zones may be exposed to completely different fluidic samples.

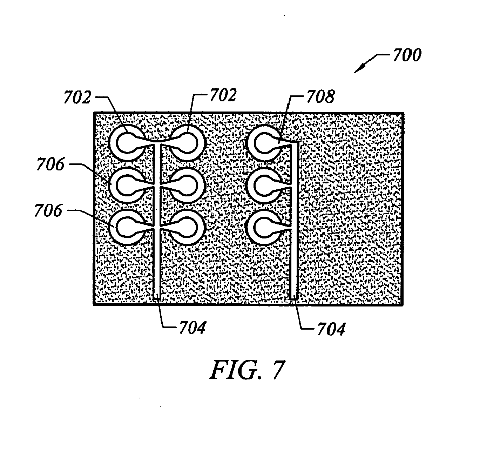

Figure 7 shows an additional embodiment of the vertical tube configuration. Instead of taking fluid back up through the concentric tube, a new fluidic template 700 is attached to a sensor surface. This template 700 has formed wells 702 for the tube delivery, but a horizontal drain network 704 that allows a common drain for the interaction zones. The ring 706 serves as a seal for the vertical delivery tube (which is perpendicular to the plane of the page). The channels 704 serve to efficiently sweep the delivered fluid from the interaction zone and out to a common waste. The decreasing channel diameter 708 accelerates the fluid from the interaction zone and also serves to limit crosstalk between zones.

The invention and its various embodiments allow one to easily prepare multiple sensing surfaces, measure multiple interactions for multiple samples simultaneously, and obtain accurate data for kinetic measurements. The invention minimizes the mixing of the sample with the flowing buffer and maintains the fidelity of the sample profile and transport through the sensing areas. Since the sensor surfaces are prepared in the instrument, the user can readily determine how much of the interaction species has been immobilized, allowing for better measurement control. This also eliminates the need for additional instrumentation, such as a printer, for making the sensor surface. In addition, multiple interaction flow paths may be prepared simultaneously, reducing the time spent in preparation and improving the throughput of the instrument. The multiplexing scheme takes advantage of parallel fluidic flow and the rotation of the flow paths in one embodiment. In another embodiment, the system allows individual samples to be measured against individual interaction species, allowing the largest degree of sample throughput possible.

The foregoing description, for purposes of explanation, used specific nomenclature to provide a thorough understanding of the invention. However, it will be apparent to one skilled in the art that specific details are not required in order to practice the invention. Thus, the foregoing descriptions of specific embodiments of the invention are presented for purposes of illustration and description. They are not intended to be exhaustive or to limit the invention to the precise forms disclosed; obviously, many modifications and variations are possible in view of the above teachings. The embodiments were chosen and described in order to best explain the principles of the invention and its practical applications, they thereby enable others skilled in the art to best utilize the invention and various embodiments with various modifications as are suited to the particular use contemplated. It is intended that the following claims and their equivalents define the scope of the invention.

The disclosures in United States patent application No. 10/987,991, from which this application claims priority, and in the abstract accompanying this application are incorporated herein by reference.

## Claims

1. A method comprising:
providing an interaction species fluidic router (300) in fluidic communication with a substrate (305); and
routing a set of interaction species samples through said interaction species fluidic router to produce a coated sensor surface with multiple reaction zones (202) corresponding to said interaction species samples.

2. A method as claimed in claim 1 further comprising:
removing said interaction species fluidic router (300) from said substrate (305);
connecting a multi-sample fluidic router (310) to said substrate; and
directing a plurality of fluidic samples through said multi-sample fluidic router to said multiple reaction zones.

3. A method as claimed in claim 2 wherein connecting includes connecting said multi-sample fluidic router (310) in an orthogonal orientation to the orientation of said interaction species fluidic router (300).

4. A method as claimed in claim 2 or 3 wherein directing includes directing a plurality of fluidic samples from a single source to said multiple reaction zones.

5. A method as claimed in claim 2 or 3 wherein directing includes directing a plurality of fluidic samples from multiple sources to said multiple reaction zones.

6. A method as claimed in any preceding claim wherein routing includes routing a set of disparate interaction species samples through said interaction species fluidic router (300) to produce a coated sensor surface with multiple disparate reaction zones (202) corresponding to said disparate interaction species samples.

7. A fluidic system (100) for biomolecular interaction measurements, comprising:
a substrate (106) with multiple reaction zones; and
a multi-sample fluidic router (102) to route a plurality of fluidic samples to said multiple reaction zones of said substrate.

8. A fluidic system as claimed in claim 7 wherein said multiple reaction zones define a single interaction species for interaction with a plurality of disparate fluidic samples.

9. A fluidic system as claimed in claim 7 wherein said multiple reaction zones include multiple interaction species for interaction with a single fluidic sample.

10. A fluidic system of claim 7 wherein said multiple reaction zones include multiple interaction species for interaction with a plurality of disparate fluidic samples.

11. A fluidic system as claimed in any of claims 7 to 10 wherein said multi-sample fluidic router (102) includes a fluidic port array with vertical ports.

12. A fluidic system as claimed in any of claims 7 to 11 wherein said multi-sample fluidic router (102) includes a fluidic port array with horizontal ports.

13. A fluidic system as claimed in any of claims 7 to 12 wherein said multi-sample fluidic router (102) includes a set of fluid transport tubes corresponding to said multiple reaction zones, wherein each fluidic transport tube includes concentric tubes defining an ingress fluidic path and egress fluidic path.
